# EUROPEAN PATENT APPLICATION

(11) **EP 4 529 789 A1**
(43) Date of publication of application: **02.04.2025**
(21) Application number: 23201032.2
(22) Date of filing: 29.09.2023
(51) Int. Cl.: A24F 40/50, A24F 40/53

(54) **AEROSOL GENERATING APPARATUS AND METHOD OF CONTROLLING AN AEROSOL GENERATING APPARATUS**

(71) Applicant: IMPERIAL TOBACCO LIMITED, Bristol BS3 2LL (GB)
(72) Inventor: The designation of the inventor has not yet been filed
(74) Representative: Mewburn Ellis LLP

(57) **Abstract**

The invention relates to an aerosol generating apparatus (1) for delivering an aerosol to a user, comprising a storage portion adapted to store a precursor, an aerosol generating unit (4) configured to generate the aerosol from the precursor, a detector unit (18, 60) configured to detect a start of a puff and to generate a corresponding puff signal, and an electrical circuitry configured to start the operation of the aerosol generating unit (4) upon receiving the puff signal, and end the operation of the aerosol generating unit (4) before the puff ends.

## Description

### FIELD

The present disclosure relates to an aerosol generating apparatus, to a method of controlling an aerosol generating apparatus, and to an electrical circuitry for an aerosol generating apparatus.

### BACKGROUND

A typical aerosol generating apparatus may comprise a power supply, an aerosol generating unit that is driven by the power supply, an aerosol precursor, which in use is aerosolised by the aerosol generating unit to generate an aerosol, and a delivery system for delivery of the aerosol to a user.

A drawback with known aerosol generating apparatuses is the accumulation of condensed aerosol in the aerosol generating apparatus. This may lead to leakage - for example into the mouth of a user. In spite of the effort already invested in the development of aerosol generating apparatuses/systems further improvements are desirable.

### SUMMARY

The present disclosure provides an aerosol generating apparatus for delivering an aerosol to a user, e.g. for inhalation by the user. The aerosol generating apparatus comprises an aerosol generating unit configured to generate the aerosol from a precursor, a detector unit configured to detect a start of a puff by the user of the aerosol generating apparatus and to generate a corresponding puff signal. Optionally, the aerosol generating apparatus further comprises a storage portion adapted to store the precursor.

In some examples, the aerosol generating apparatus further comprises an electrical circuitry that is configured to start the operation of the aerosol generating unit upon receiving the puff, and to end the operation of the aerosol generating unit before the puff ends.

An advantage of the invention is that the user draws or inhales longer on the aerosol generating apparatus than the aerosol generating unit is operated for the generation of the aerosol. This means that there is a period at the end of the puff when the user draws on the aerosol generating apparatus and the aerosol generating unit is not operated for the generation of the aerosol from the precursor. During this period, the user basically draws the aerosol that has been previously generated (and still is within the aerosol generating apparatus) and/or draws the aerosol that is generated by a remaining heat of the aerosol generating unit. This prevents or reduces the likelihood that aerosol remains within the aerosol generating apparatus. The remaining aerosol could condensate within the aerosol generating apparatus when the operation of the aerosol generating apparatus coincides with the puff of the use. Thus, the invention prevents or reduces the likelihood that the condensate aerosol may leak from the aerosol generating apparatus. For example, the condensate aerosol may be sucked into the mouth of the user during a subsequent puff.

In other words, by ending the operation of the aerosol generating apparatus before the user ends the puff, aerosol remaining within the aerosol generating apparatus can be removed from the aerosol generating apparatus. This remaining aerosol can result in the accumulation of condensate aerosol. In this way, leakage of condensate aerosol can be reduced or prevented.

The detector unit may include sensors, meters, and/or electronic components. For example, parts of the detector unit may be implemented by electronic circuitry so that the detector unit or a part thereof may be a portion of the electronic circuitry. The detector unit is configured to detect when a user starts drawing on the aerosol generating apparatus for inhaling the aerosol. The detector unit generates the puff signal when the user starts drawing on the aerosol generating apparatus. The point in time when the puff signal is generated may mark the start of puff of the user.

The detector unit and the aerosol generating unit may be in data communication with the electrical circuitry. For example, the detector unit and the aerosol generating unit are connected to the electronic circuitry by one or more wires. The electrical circuitry can receive the puff signal from the detector unit and/or can control the aerosol generating unit, for example by controlling the power that is supplied to the aerosol generating unit. The control of the aerosol generator unit by the electrical circuitry may include starting the operation of the aerosol generating unit and/or ending the operation of the aerosol generating unit (this may also be considered as activating and de-activating the aerosol generating unit, respectively). For example, the electronic circuitry may control the supply of power to the aerosol generating unit for controlling the operation of the aerosol generating unit. The operation of the aerosol generating unit may be considered a period during which the aerosol is generated from the precursor.

Further, the detector unit may generate the puff signal as long as the user is inhaling. Before and/or after the puff, the detector unit may not generate the puff signal. In other words, as long as the user is inhaling, the puff signal is being generated in some examples. Optional embodiments how the detector unit can determine that the user makes a puff are described further below.

The detector unit may continuously generate the puff signal as long as the user is drawing on the aerosol generating apparatus. In this case, the initial reception of the puff signal indicates the start of the puff, and the termination of the puff signal indicates the end of the puff. Alternatively, the puff signal may include a plurality of intermediate signals which are periodically sent out by the detector unit (e.g., every 5 ms, 10 ms, or 20 ms) if the detector unit determines that a puff is made. In this case, the electrical circuitry changes the control of the aerosol generator unit (e.g., whether to operate at the aerosol generator unit are not) in line with the periodically expected intermediate signals. The electrical circuitry maintains the control of the aerosol generating unit between the intermediate signals. Further, the detector unit may send the puff signal once the start of a puff is detected. The puff signal may be valid as long as the detector unit sends out a further signal (e.g., a puff end signal) indicating the end of the puff once the detector unit determines that the puff ended.

The electrical circuitry can be configured to end the operation of the aerosol generating unit (e.g., generation of the aerosol from the precursor such as by heating of the aerosol generating unit) before the puff ends as indicated by the puff signal. In one, although the electrical circuitry still determines that user puffs (e.g., by receiving the puff signal) and would - with prior art apparatuses - control the aerosol generator unit to generate the aerosol, the electrical circuitry controls the aerosol generating unit to end the generation of the aerosol (e.g., by stopping the supply of power to the aerosol generating unit). Optional embodiments when exactly to end operation of the aerosol generating unit (end the generation of a result) are described further below.

In some examples, the detector unit includes an airflow sensor for measuring a flow rate of the aerosol and/or of air flowing through aerosol generating apparatus. Optionally, the electrical circuitry is further configured to determine an expected end of the puff based on the determined flow rate. Further optionally, the electrical circuitry is configured to end the operation of the aerosol generating unit prior to the expected end of the puff.

In this way, the aerosol generating apparatus includes the functionality to determine, calculate, estimate, and/or compute an expected end of the user's puff. This is done to determine when to stop the operation of the aerosol generating unit, e.g., when to end the generation of the aerosol from the precursor. In other words, the electrical circuitry controls the duration of the generation of the aerosol based on the expected end or duration of the puff.

The electrical circuitry may be configured to predict the end of the puff. In one example, the prediction is made using the measurement of the an airflow sensor which can measure the flow rate of the aerosol (e.g., an aerosol downstream of the aerosol generating unit) and/or air flowing through the aerosol generating apparatus (e.g., a flow of air upstream of the aerosol generating unit). In other words, the airflow sensor may be arranged upstream and/or downstream of the aerosol generating unit. The airflow sensor may be arranged within and/or in fluid communication with a flow path (e.g., an internal passageway (channel or lumen) of the aerosol generating apparatus through which the air/aerosol flows).

The airflow sensor may include any type of sensor that is configured to measure the flow rate of a gas or aerosol. The airflow sensor may be configured for a flow measurement which is the quantification of bulk fluid movement. The airflow sensor may include a flowmeter. The airflow sensor may be configured to measure a mass flow rate. The airflow sensor may include ultrasonic flow meters, thermal mass flowmeters, Coriolis mass flowmeters, or mass flow controllers. The airflow sensor may be a pressure-based meter, an optical flowmeter, and/or a thermal mass flowmeter.

The airflow sensor may be in data communication with the electrical circuitry and/or may be connected to the electrical circuitry by one or more wires. The airflow sensor may generate a flow signal which is indicative of the strength of the flow rate of the aerosol/air flowing in the aerosol generating apparatus.

The flow signal may be sent to the electrical circuitry which may analyse the flow signal (i.e., the flow rate) for determining the expected end of the puff. The determination of the expected end of the puff may not be direct. For example, the electrical circuitry may only determine when to end the generation of the aerosol. However, the end of the generation of the aerosol relates to the expected puff time or the expected end of the puff so that the electrical circuitry may indirectly determine the expected end of the puff. Optional embodiments of the control executed by the electrical circuitry are described further below.

For example, the electrical circuitry stores and/or determines a preset timeslot between the expected end of the puff and the point of time when the electrical circuitry controls the aerosol generating unit to stop the generation of the aerosol from the precursor. For example, the present timeslot corresponds to the time during which the user still draws on the aerosol generating apparatus, but no aerosol is generated by the aerosol generating unit (e.g., the aerosol generating unit is not activated or powered). The preset timeslot may be set so short that the user still draws aerosol at the end of the puff. So, the preset timeslot may be chosen so that, at the end of the puff, aerosol is still inhaled by the user. Thus, the preset timeslot is chosen so that not all aerosol that has previously generated and is within the aerosol generating apparatus has already been inhaled. On the other hand, the preset timeslot may be set so long that a sufficient amount of the aerosol that is generated after the aerosol generating unit stopped operating and/or the aerosol that is present within the aerosol generating apparatus is inhaled during the preset timeslot. In other words, the preset timeslot should be chosen sufficiently long that a sufficient amount of aerosol can still be inhaled, and the condensation of aerosol is sufficiently reduced.

The preset timeslot may be determined in advance (e.g., a factory setting) and/or may be set and/or changed by the user in view of the above-mentioned criteria. Further, the preset timeslot may be specific to the aerosol generating apparatus and/or may be specific to the type of the aerosol generating apparatus and, therefore may be a factory setting for all the general aerosol generating apparatus of the same type.

In some examples, the electrical circuitry is further configured to determine a peak of the flow rate and end the operation of the aerosol generating unit at a predetermined time period after the peak of the flow rate.

In this way, the aerosol generating apparatus may estimate the expected end of the puff. In other words, a peak of the flow rate can be indicative of the expected end of the puff. For example, the period between the peak of the flow rate and the end of the puff is expected to be approximately constant for all puffs of the same user. So, by appropriately setting the predetermined time period, the generation of the aerosol can be terminated before the puff ends, for example by stopping the operation of the aerosol generating unit. The same criteria as described in connection with the preset timeslot may be applied for setting or determining the predetermined time period. The setting of the predetermined time period corresponds to an indirect determination of the end of the puff of the user.

For example, the preset timeslot corresponds to time interval between the peak of the flow rate and the expected end of the puff minus the predetermined time period.

The inventors have found out that the flow rate of a puff increases at the beginning of the puff, peaks, and then the flow rate decreases until the end of the puff. In other words, it has been found that there usually is only a single peak of the flow rate during one puff and the time interval between the peak of the flow rate and the end of the puff is approximately constant over many puffs. The peak of the flow rate can be easily determined and is indicative of the end of the user's puff. For determining the peak of the flow rate, conventional techniques for determining the peak of a graph may be used. This functionality of determining the peak of the flow rate may be executed by the electrical circuitry.

In some examples, the electrical circuitry is further configured to determine a rate of decrease in the flow rate and to end the operation of the aerosol generating unit after the rate of decrease in the flow rate is above a predetermined threshold.

In this way, the aerosol generating apparatus may estimate the expected end of the puff. In other words, the rate of decrease of the flow rate can be indicative of the end of the expected puff. The inventors found out that the rate in the decrease in the flow rate increases steadily after the peak of the flow rate. In particular, the decrease in the flow rate is larger at the end of the puff. Again, the decrease in the flow rate is approximately constant over many puffs. Thus, the rate of the decrease in the flow rate being above a predetermined threshold can be a reliable criterion for predicting the end of the puff. For example, reaching the predetermined threshold is expected to be a certain timeslot before the expected end of the puff. In other words, with this approach, the expected end of the puff is only indirectly determined. The same criteria as described in connection with the preset timeslot may be applied for setting or determining the predetermined threshold. In other words, the predetermined threshold is chosen that a sufficient amount of aerosol within the aerosol generator apparatus is inhaled by the user after the aerosol generating unit stops the generation of the aerosol.

In some examples, the predetermined time period and/or the predetermined threshold are pre-stored values.

In this way, the predetermined time period and the predetermined threshold may be factory settings which can be determined by an analysis of the flow rate profiles of a plurality of users of the aerosol generating apparatus. This has the advantage that the user is not required to set the predetermined time period and/or the predetermined threshold. So, the aerosol generating apparatus can be more easily used. Further, the aerosol generating apparatus does not need to include a functionality for automatically determining the best predetermined threshold and/or predetermined time period.

In some examples, the electrical circuitry is further configured to calculate a rolling average of the flow rate over the last n puffs for setting the predetermined time period and/or the predetermined threshold, n being an integer > 1.

In this way, the electrical circuitry may be configured to automatically set the predetermined time period and/or the predetermined threshold. So, no user interaction is required for fine tuning the predetermined time period and/or the predetermined threshold. This has the advantage that the predetermined time period and/or the predetermined threshold can be more accurately set for a respective user of the aerosol generating apparatus.

For example, the electrical circuitry is configured to execute a program, algorithm, or the like for calculating an average flow rate profile of a user puff by averaging the flow rate profiles of the previous n puffs. Based on the average flow rate profile, the electrical circuitry may be configured to set the predetermined time period and/or the predetermined threshold. For example, the predetermined time period and/or the predetermined threshold are set so that the operation of the aerosol generating unit ends at the expected end of the puff minus the preset timeslot. Alternatively or additionally, the electrical circuitry may be configured to estimate the amount of aerosol that is generated and calculates the end of the operation of the aerosol generating unit in view of the expected amount of aerosol that is generated and/or inhaled during the time the user inhales but the aerosol generating unit is no longer operated.

In a further optional embodiment, the electrical circuitry either includes an artificial intelligence or can be connected to an artificial intelligence for determining predetermined time period and/or the predetermined threshold.

In some examples, the electrical circuitry is further configured to only end the operation of the aerosol generating unit if the flow rate is below a predetermined level.

In this way, a failsafe mechanism is provided for avoiding that the operation of the generating unit (i.e., the generation of aerosol) is stopped too early, for example because of a miscalculation of the peak of the flow rate or the decrease in the flow rate.

The predetermined level may be set sufficiently low so that the operation of the aerosol generating unit is not stopped too early. The predetermined level may be a factory setting which is determined by analysing a plurality of puff profiles of various users.

In some examples, the detector unit includes a pressure sensor configured to generate the puff signal when a pressure of the aerosol and/or an air flowing through aerosol generating apparatus is below a predetermined pressure threshold. Optionally, the electrical circuitry is further configured to end the operation of the aerosol generating unit after a predetermined time interval after receiving the puff signal.

In this way, a simple and yet reliable design of the aerosol generator apparatus can be provided. For example, the pressure sensor used may not be so sensitive as the airflow sensor described above because it only needs to distinguish between an air pressure below the predetermined pressure threshold and above the predetermined pressure threshold. A puff may result in the reduction of the pressure within the flow path.

The pressure sensor may be capacitive and can use a diaphragm and pressure cavity to create a variable capacitor to detect strain due to applied pressure - the capacitance decreases or increases as pressure deforms the diaphragm. Since the pressure sensor only needs to detect whether the pressure is below the predetermined pressure threshold, the pressure sensor may have a simple design and/or can work reliably. The predetermined pressure threshold may be set so that air and/or aerosol pressures detected by the pressure sensor to be below the predetermined pressure threshold indicate a puff or the draw by the user.

The pressure sensor may be arranged within and/or in fluid communication with a flow path (e.g., an internal passageway (channel or lumen) of the aerosol generating apparatus through which the air/aerosol flows). The pressure sensor may be configured to measure a pressure of the aerosol and/or the air that flows through the aerosol generator apparatus. For example, the pressures sensor can measure the pressure of the aerosol (e.g., an aerosol downstream of the aerosol generating unit) and/or the pressure of the air flowing through the aerosol generating apparatus (e.g. a flow of air upstream of the aerosol generating unit). The pressure sensor may be configured to generate the puff signal if the measured pressure is below the predetermined pressure threshold. In some embodiments, the pressure sensor does not measure an absolute pressure, but merely detects whether the pressure is below the predetermined pressure threshold.

The electrical circuitry is in data communication with the pressure sensor and/or can be electrically connected to the pressure sensor using one or more wires. The electrical circuitry is configured to stop the operation of the aerosol generating unit (e.g., stop heating the precursor) after a predetermined time interval after receiving the puff signal. The time of receipt of the puff signal indicates the start of the puff so that the electrical circuitry is configured to end the heating of the precursor before the puff ends if the predetermined time interval is appropriately set.

The predetermined time interval may correspond to the average time of the puff minus the preset timeslot as described above. Thus, this embodiment is based on the realisation that the puff of a user has generally the same length. Thus, without requiring further analysis of the flow rate of the user's puff, the electrical circuitry can end the operation of the aerosol generating unit before the puff ends. As described above, the predetermined time interval can be a factory setting, can be manually set by the user, and/or the electrical circuitry can include means for determining the average length of the puff.

The above-described embodiments can be optionally used with aerosol generating apparatuses that have a liquid as a precursor. The aerosol may be generated by heating the liquid. In this case, the aerosol generating unit may only be operated or powered when a puff is detected (e.g., the puff signal is generated).

In some examples, e.g., for aerosol generating apparatuses using tobacco as a precursor, the detector unit is further configured to determine the power supplied to the aerosol generating unit and generate the puff signal based on the power supplied to the aerosol generating unit.

In this way, the aerosol generating unit may be controlled to provide a constant temperature by appropriately controlling the power supplied to aerosol generating unit. An increase in the power supplied to the aerosol generating unit can indicate the presence of a puff because drawing on the aerosol generating apparatus by the user cools the air aerosol generating unit. As a result, more power is required for maintaining a preset temperature. Thus, in this embodiment, no airflow sensor or pressure sensor is required but the puff is detected by monitoring the power supplied to aerosol generating unit.

For example, if the power supplied to the aerosol generating unit is above a predetermined threshold, optionally for a predetermined time period, it is assumed that the puff is present, and the detector unit generates the puff signal. With this embodiment, the detector unit may be a part or portion of the electrical circuitry. The detector unit may be configured to monitor and/or measure the current and/or the voltage supplied to the aerosol generating unit and/or a resistance of the aerosol generating unit. These monitored and/or measured values can be used for calculating the power supplied to the aerosol generating unit. The detector unit may include a voltmeter, the amperemeter, and/or a meter for measuring the resistance of the aerosol generating unit.

In some examples, the electrical circuitry is configured to control the power supplied to the aerosol generating unit by using pulse width modulation (PWM). Optionally, the detector unit is configured generate the puff signal based on a ratio of on-times compared to off-times in the PWM.

In this way, the puff signal can be generated with available information when controlling the aerosol generating unit using PWM. An additional airflow sensor and/or pressure sensor is not required. In this embodiment, the detector unit may be a part of the electrical circuitry.

Pulse-width modulation (PWM) is a method of controlling the average power delivered from the power supply to the aerosol generating unit. The average value of voltage (and current) fed to the aerosol generating unit is controlled by switching the supply between 0 and 100% at a rate faster than it takes the aerosol generating unit to change significantly, e.g., the generated heat. The longer the switch is on, the higher the total power supplied to the load. The electrical circuitry may be configured to control the switch for controlling the power and, therefore, the temperature of the aerosol generating unit. The on-times are the time intervals of the PWM control during which power is supplied to the aerosol generating unit. The off-times are time intervals of the PWM control during which no power is supplied to the aerosol generating unit. So, in cases of high-power supply to the aerosol generating unit, the ratio of the on-times compared to the off-times is higher and vice versa.

For example, a particular ratio of on-times to off-times indicates a power supply to the aerosol generating apparatus that corresponds to maintaining a constant temperature when the user does not draw on the aerosol generating apparatus. An increase in the ratio of the on-times to off-times may indicate that the user makes the puff, for example if this increased ratio of on-times to off-times is maintained over a certain period of time. In this case, the detector unit may generate the puff signal. The generation of the puff signal may be stopped if the ratio of the on-times to off-times returns to the particular ratio of on-times to off-times indicating the maintenance of a constant temperature when the user does not draw on the aerosol generating apparatus.

In some examples, the electrical circuitry is configured to set the predetermined time interval as an average puff length minus a preset timeslot.

In this way, the duration of the timeslot indicates how long before the puff ends, the operation of the aerosol generating unit is stopped. Thus, the timeslot provides how long the user draws on the aerosol generating apparatus without the aerosol generating unit being in operation. Thus, by appropriately setting the timeslot, the user does not notice that the aerosol generating unit was not in operation because a sufficient amount of aerosol was already present in the aerosol generating apparatus and/or the remaining heat of the aerosol generating unit provided a sufficient amount of aerosol. On the other hand, the condensation of aerosol within the aerosol generating apparatus can be reliably reduced. The duration of the timeslot may depend on the user's average puff (e.g., on the flow rate profile at the end of the puff).

In some examples, the electrical circuitry is configured to set the average puff length by averaging a plurality of puffs in a learning mode and/or by calculating a rolling average of the puff length over the last n puffs, n being an integer > 1.

In this way, the average puff length can be adjusted to the respective user of the aerosol generating apparatus.

The learning mode may be used prior to using the aerosol generating apparatus and/or when the user believes that the average puff length and/or the timeslot are not appropriately set. The learning mode may include drawing on the aerosol generating apparatus for n times (n being an integer > 1, e.g. 5, 10, 15) without the aerosol generating apparatus being operated. The learning mode may be solely provided for measuring the puff length for each puff and calculating an average puff length. For example, as described above, the duration of the puff is determined by monitoring the receipt of the puff signal which is generated by the detector unit in the various examples described herein. During normal operation of the aerosol generating apparatus, i.e., not in the learning mode, the length of the puff may not be recorded. For the learning mode, the inventors' finding is used that the average puff length is basically constant overtime. The learning mode may also be provided for setting the predetermined time period, the predetermined threshold, and/or the preset timeslot using the methods described herein.

Alternatively, when using the rolling average, the electrical circuitry measures the length of the puff for each puff and calculates an average puff length on the basis of the last n puff lengths. So, in this case, there may not be a learning mode and the average puff length is continuously adjusted. This may provide a more accurate and updated average puff length compared to using the learning mode.

In some examples, the aerosol generating apparatus further comprises an input device for manually changing the preset time interval, predetermined time period, the predetermined threshold, and/or the timeslot.

In this way, the average time interval and/or the timeslot can be manually adjusted by the user. So, the electrical circuitry does not need to include functionalities for measuring and averaging the puff length resulting in a more simplified setup of the electrical circuitry.

The input device may include buttons, dials, switches and/or a touchscreen with which the preset time interval, predetermined time period, the predetermined threshold, and/or the timeslot can be set and/or changed. The aerosol generating apparatus may further include a user interface or display for displaying the currently set preset time interval, predetermined time period, the predetermined threshold, and/or the timeslot. The electrical circuitry may be in data communication with input device and the user interface/display. The electrical circuitry may control the user interface/display and/or the electrical circuitry may receive signals from the device indicative of the user input.

The present disclosure may provide a method of controlling an aerosol generating apparatus for delivering an aerosol to a user. The method comprises the steps of detecting a start of a puff of the user and generating a corresponding puff signal, starting the operation of an aerosol generating unit for generating the aerosol from the precursor as soon as when the puff signal is generated, and ending the operation of the aerosol generating unit before the puff ends.

The method may implement any one or more features disclosed herein. For example, the method may include steps which correspond to the functionalities executed by the electrical circuitry.

In some examples, the step of detecting the puff includes measuring a flow rate of the aerosol and/or of air flowing through aerosol generating apparatus, wherein the method further comprises determining an expected end of the puff based on the determined flow rate and ending the operation of the aerosol generating unit prior to the expected end of the puff.

The present disclosure may provide an electrical circuitry and/or a computer program configured to cause an aerosol generating apparatus to perform any method or method step disclosed herein. A computer readable medium comprising the computer program is also disclosed.

For example, the present disclosure may provide an electrical circuitry for an aerosol generating apparatus for delivering an aerosol to a user, the electrical circuitry being configured to execute the following steps: receiving a puff signal indicating a start of a puff, generating an operation signal for starting the operation of an aerosol generating unit for generating the aerosol from the precursor upon receiving the puff signal, and ending the generation of the operation signal before the puff signal ends.

The preceding summary is provided for purposes of summarizing some examples to provide a basic understanding of aspects of the subject matter described herein. Accordingly, the above-described features should not be construed to narrow the scope or spirit of the subject matter described herein in any way. Moreover, the above and/or proceeding examples may be combined in any suitable combination to provide further examples, except where such a combination is clearly impermissible or expressly avoided. Other features, aspects, and advantages of the subject matter described herein will become apparent from the following text and the accompanying drawings.

### BRIEF DESCRIPTION OF THE FIGURES

Aspects, features and advantages of the present disclosure will become apparent from the following description of examples in reference to the appended drawings in which like numerals denote like elements.
**Fig. 1A** is a block system diagram showing an example aerosol generating apparatus.
**Fig. 1B** is a block system diagram showing a further example aerosol generating apparatus.
**Fig. 2** is a block system diagram showing an example implementation of the apparatus of Fig. 1, where the aerosol generating apparatus is configured to generate aerosol from a liquid precursor.
**Figs. 3A and 3B** are schematic diagrams showing an example implementation of the apparatus of Fig. 2.
**Fig. 4** is a block system diagram showing an example implementation of the apparatus of Fig. 1, where the aerosol generating apparatus is configured to generate aerosol from a solid precursor.
**Fig. 5** is a schematic diagram showing an example implementation of the apparatus of Fig. 4.
**Fig. 6** is a block system diagram showing an example system for managing an aerosol generating apparatus.
**Fig. 7** is a diagram showing various graphs of flow rates, wherein each graph indicates an average flow rated averaged over plurality of puffs and a plurality of users.
**Fig. 8** is a block diagram indicating steps of a method for controlling an aerosol generating apparatus for delivering an aerosol to a user for inhalation by the user.

### DETAILED DESCRIPTION OF EMBODIMENTS

Before describing several examples implementing the present disclosure, it is to be understood that the present disclosure is not limited by specific construction details or process steps set forth in the following description and accompanying drawings. Rather, it will be apparent to those skilled in the art having the benefit of the present disclosure that the systems, apparatuses and/or methods described herein could be embodied differently and/or be practiced or carried out in various alternative ways.

Unless otherwise defined herein, scientific and technical terms used in connection with the presently disclosed inventive concept(s) shall have the meanings that are commonly understood by those of ordinary skill in the art, and known techniques and procedures may be performed according to conventional methods well known in the art and as described in various general and more specific references that may be cited and discussed in the present specification.

Any patents, published patent applications, and non-patent publications mentioned in the specification are hereby incorporated by reference in their entirety.

All examples implementing the present disclosure can be made and executed without undue experimentation in light of the present disclosure. While particular examples have been described, it will be apparent to those of skill in the art that variations may be applied to the systems, apparatus, and/or methods and in the steps or in the sequence of steps of the methods described herein without departing from the concept, spirit, and scope of the inventive concept(s). All such similar substitutions and modifications apparent to those skilled in the art are deemed to be within the spirit, scope, and concept of the inventive concept(s) as defined by the appended claims.

The use of the term "a" or "an" in the claims and/or the specification may mean "one," as well as "one or more," "at least one," and "one or more than one." As such, the terms "a," "an," and "the," as well as all singular terms, include plural referents unless the context clearly indicates otherwise. Likewise, plural terms shall include the singular unless otherwise required by context.

The use of the term "or" in the present disclosure (including the claims) is used to mean an inclusive "and/or" unless explicitly indicated to refer to alternatives only or unless the alternatives are mutually exclusive. For example, a condition "A or B" is satisfied by any of the following: A is true (or present) and B is false (or not present), A is false (or not present) and B is true (or present), and both A and B are true (or present).

As used in this specification and claim(s), the words "comprising, "having," "including," or "containing" (and any forms thereof, such as "comprise" and "comprises," "have" and "has," "includes" and "include," or "contains" and "contain," respectively) are inclusive or open-ended and do not exclude additional, unrecited elements or method steps.

Unless otherwise explicitly stated as incompatible, or the physics or otherwise of the embodiments, examples, or claims prevent such a combination, the features of examples disclosed herein, and of the claims, may be integrated together in any suitable arrangement, especially ones where there is a beneficial effect in doing so. This is not limited to only any specified benefit, and instead may arise from an "ex post facto" benefit. This is to say that the combination of features is not limited by the described forms, particularly the form (e.g. numbering) of example(s), embodiment(s), or dependency of claim(s). Moreover, this also applies to the phrase "in one embodiment," "according to an embodiment," and the like, which are merely a stylistic form of wording and are not to be construed as limiting the following features to a separate embodiment to all other instances of the same or similar wording. This is to say, a reference to 'an,' 'one,' or 'some' embodiment(s) may be a reference to any one or more, and/or all embodiments, or combination(s) thereof, disclosed. Also, similarly, the reference to "the" embodiment may not be limited to the immediately preceding embodiment. Further, all references to one or more embodiments or examples are to be construed as non-limiting to the claims.

The present disclosure may be better understood in view of the following explanations, wherein the terms used that are separated by "or" may be used interchangeably:
As used herein, an **"aerosol generating apparatus"** (or **"electronic(e)-cigarette"**) may be an apparatus configured to deliver an aerosol to a user for inhalation by the user. The apparatus may additionally/alternatively be referred to as a "smoking substitute apparatus", if it is intended to be used instead of a conventional combustible smoking article. As used herein a combustible "smoking article" may refer to a cigarette, cigar, pipe or other article, that produces smoke (an aerosol comprising solid particulates and gas) via heating above the thermal decomposition temperature (typically by combustion and/or pyrolysis). An aerosol generated by the apparatus may comprise an aerosol with particle sizes of 0.2 - 7 microns, or less than 10 microns, or less than 7 microns. This particle size may be achieved by control of one or more of: heater temperature (e.g., a temperature of an aerosol generating unit); cooling rate as the vapour condenses to an aerosol; flow properties including turbulence and velocity. The generation of aerosol by the aerosol generating apparatus may be controlled by an input device. The input device may be configured to be user-activated and may for example include or take the form of an actuator (e.g., actuation button), an airflow sensor, and/or a pressure sensor.

Each occurrence of the aerosol generating apparatus being caused to generate aerosol for a period of time (which may be variable) may be referred to as an **"activation"** or **"operation"** of the aerosol generating apparatus. The aerosol generating apparatus may be arranged to allow an amount of aerosol delivered to a user to be varied per activation (as opposed to delivering a fixed dose of aerosol), e.g. by activating the aerosol generating unit of the aerosol generating apparatus for a variable amount of time, e.g. based on the strength/duration of a draw of a user through a flow path of the apparatus (to replicate an effect of smoking a conventional combustible smoking article).

The aerosol generating apparatus may be portable. As used herein, the term **"portable"** may refer to the apparatus being for use when held by a user.

As used herein, an **"aerosol generating system"** may be a system that includes an aerosol generating apparatus and optionally other circuitry/components associated with the function of the apparatus, e.g., one or more external devices and/or one or more external components (here "external" is intended to mean external to the aerosol generating apparatus).

As used herein, an "external device" and "external component" may include one or more of a: a charging device, a mobile device (which may be connected to the aerosol generating apparatus, e.g., via a wireless or wired connection); a networked-based computer (e.g., a remote server); a cloud-based computer; any other server system.

An example aerosol generating system may be a system for managing an aerosol generating apparatus. Such a system may include, for example, a mobile device, a network server, as well as the aerosol generating apparatus.

As used herein, an **"aerosol"** may include a suspension of precursor, including as one or more of: solid particles; liquid droplets; gas. Said suspension may be in a gas including air. An aerosol herein may generally refer to/include a vapour. An aerosol may include one or more components of the precursor. As used herein, a **"precursor"** may include one or more of a: liquid; solid; gel; loose leaf material; other substance. The precursor may be processed by the aerosol generating unit of an aerosol generating apparatus to generate an aerosol. The precursor may include one or more of: an active component; a carrier; a flavouring. The active component may include one or more of nicotine; caffeine; a cannabidiol oil; a non-pharmaceutical formulation, e.g., a formulation which is not for treatment of a disease or physiological malfunction of the human body. The active component may be carried by the carrier, which may be a liquid, including propylene glycol and/or glycerine. The term "flavouring" may refer to a component that provides a taste and/or a smell to the user. The flavouring may include one or more of: Ethylvanillin (vanilla); menthol, Isoamyl acetate (banana oil); or other. The precursor may include a substrate, e.g., reconstituted tobacco to carry one or more of the active component; a carrier; a flavouring.

As used herein, a **"storage portion"** may be a portion of the apparatus adapted to store the precursor. It may be implemented as fluid-holding reservoir or carrier for solid material depending on the implementation of the precursor as defined above. The storage portion may also encompass a cavity for receiving the precursor, e.g., tobacco stick.

As used herein, a **"flow path"** may refer to a path or enclosed passageway through an aerosol generating apparatus, e.g., for delivery of an aerosol to a user. The flow path may be arranged to receive aerosol from an aerosol generating unit. When referring to the flow path, upstream and downstream may be defined in respect of a direction of flow in the flow path, e.g. with an outlet being downstream of an inlet.

As used herein, a **"delivery system"** may be a system operative to deliver an aerosol to a user. The delivery system may include a mouthpiece and a flow path.

As used herein, a **"flow"** may refer to a flow in a flow path. A flow may include aerosol generated from the precursor. The flow may include air, which may be induced into the flow path via a puff by a user. As used herein, a **"puff"** (or **"inhale"** or **"draw"**) by a user may refer to expansion of lungs and/or oral cavity of a user to create a pressure reduction that induces flow through the flow path.

As used herein, an **"aerosol generating unit"** may refer to a device configured to generate an aerosol from a precursor. The aerosol generating unit may include a unit to generate a vapour directly from the precursor (e.g., a heating system or other system) or an aerosol directly from the precursor (e.g., an atomiser including an ultrasonic system, a flow expansion system operative to carry droplets of the precursor in the flow without using electrical energy or other system). A plurality of aerosol generating units to generate a plurality of aerosols (for example, from a plurality of different aerosol precursors) may be present in an aerosol generating apparatus.

As used herein, a **"heating system"** may refer to an arrangement of at least one heating element, which is operable to aerosolise a precursor once heated. The at least one heating element may be electrically resistive to produce heat from the flow of electrical current therethrough. The at least one heating element may be arranged as a susceptor to produce heat when penetrated by an alternating magnetic field. The heating system may be configured to heat a precursor to below 300 or 350 degrees C, including without combustion.

As used herein, a **"consumable"** may refer to a unit that includes a precursor. The consumable may include an aerosol generating unit, e.g., it may be arranged as a cartomizer. The consumable may include a mouthpiece. The consumable may include an information carrying medium. With liquid or gel implementations of the precursor, e.g., an e-liquid, the consumable may be referred to as a "capsule" or a "pod" or an "e-liquid consumable". The capsule/pod may include a storage portion, e.g., a reservoir or tank, for storage of the precursor. With solid material implementations of the precursor, e.g. tobacco or reconstituted tobacco formulation, the consumable may be referred to as a "stick" or "package" or "heat-not-burn consumable". In a heat-not-burn consumable, the mouthpiece may be implemented as a filter and the consumable may be arranged to carry the precursor. The consumable may be implemented as a dosage or pre-portioned amount of material, including a loose-leaf product.

As used herein, an **"information carrying medium"** may include one or more arrangements for storage of information on any suitable medium. Examples include: a computer readable medium; a Radio Frequency Identification (RFID) transponder; codes encoding information, such as optical (e.g. a bar code or QR code) or mechanically read codes (e.g. a configuration of the absence or presents of cutouts to encode a bit, through which pins or a reader may be inserted).

As used herein **"heat-not-burn"** (or **"HNB"** or **"heated precursor"**) may refer to the heating of a precursor, typically tobacco, without combustion, or without substantial combustion (i.e. localised combustion may be experienced of limited portions of the precursor, including of less than 5% of the total volume).

As used herein, **"electrical circuitry"** may refer to one or more electrical components, examples of which may include: an Application Specific Integrated Circuit (ASIC); electronic/electrical componentry (which may include combinations of transistors, resistors, capacitors, inductors etc); one or more processors; a non-transitory memory (e.g. implemented by one or more memory devices), that may store one or more software or firmware programs; a combinational logic circuit; interconnection of the aforesaid. The electrical circuitry may be located entirely at the apparatus, or distributed between the apparatus and/or on one or more external devices in communication with the apparatus, e.g. as part of a system.

As used herein, a **"processing resource"** (or **"processor** " or **"controller"**) may refer to one or more units for processing data, examples of which may include an ASIC, microcontroller, FPGA, microprocessor, digital signal processor (DSP) capability, state machine or other suitable component. A processing resource may be configured to execute a computer program, e.g. which may take the form of machine-readable instructions, which may be stored on a non-transitory memory and/or programmable logic. The processing resource may have various arrangements corresponding to those discussed for the circuitry, e.g. on-board and/or off board the apparatus as part of the system. As used herein, any machine executable instructions, or computer readable media, may be configured to cause a disclosed method to be carried out, e.g. by an aerosol generating apparatus or system as disclosed herein, and may therefore be used synonymously with the term method.

As used herein, an **"external device"** (or **"peripheral device"**) may include one or more electronic components external to an aerosol generating apparatus. Those components may be arranged at the same location as the aerosol generating apparatus or remote from the apparatus. An external device may comprise electronic computer devices including: a smartphone; a PDA; a video game controller; a tablet; a laptop; or other like device.

As used herein, a **"computer readable medium/media"** (or **"memory"** or **"data storage"**) may include any medium capable of storing a computer program, and may take the form of any conventional non-transitory memory, for example one or more of: random access memory (RAM); a CD; a hard drive; a solid-state drive; a memory card; a DVD. The memory may have various arrangements corresponding to those discussed for the circuitry /processor. The present disclosure includes a computer readable medium configured to cause an apparatus or system disclosed herein to perform a method as disclosed herein.

As used herein, a **"communication resource"** (or **"communication interface"**) may refer to hardware and/or firmware for electronic information/data transfer. The communication resource may be configured for wired communication ("wired communication resources") or wireless communication ("wireless communication resource"). Wireless communication resources may include hardware to transmit and receive signals by radio and may include various protocol implementations e.g. the 802.11 standard described in the Institute of Electronics Engineers (IEEE) and Bluetooth^{™} from the Bluetooth Special Interest Group of Kirkland Wash. Wired communication resources may include; Universal Serial Bus (USB); High-Definition Multimedia Interface (HDMI) or other protocol implementations. The apparatus may include communication resources for wired or wireless communication with an external device.

As used herein, a **"network"** (or **"computer network"**) may refer to a system for electronic information/data transfer between a plurality of apparatuses/devices. The network may, for example, include one or more networks of any type, which may include: a Public Land Mobile Network (PLMN); a telephone network (e.g. a Public Switched Telephone Network (PSTN) and/or a wireless network); a local area network (LAN); a metropolitan area network (MAN); a wide area network (WAN); an Internet Protocol Multimedia Subsystem (IMS) network; a private network; the Internet; an intranet.

It will be appreciated that any of the disclosed methods (or corresponding apparatuses, programs, data carriers, etc.) may be carried out by either a host or client, depending on the specific implementation (i.e. the disclosed methods/apparatuses are a form of communication(s), and as such, may be carried out from either 'point of view', i.e. in corresponding to each other fashion). Furthermore, it will be understood that the terms "receiving" and "transmitting" encompass "inputting" and "outputting" and are not limited to an RF context of transmitting and receiving electromagnetic (e.g. radio) waves. Therefore, for example, a chip or other device or component for realizing embodiments could generate data for output to another chip, device or component, or have as an input data from another chip, device, or component, and such an output or input could be referred to as "transmit" and "receive" including gerund forms, that is, "transmitting" and "receiving," as well as such "transmitting" and "receiving" within an RF context.

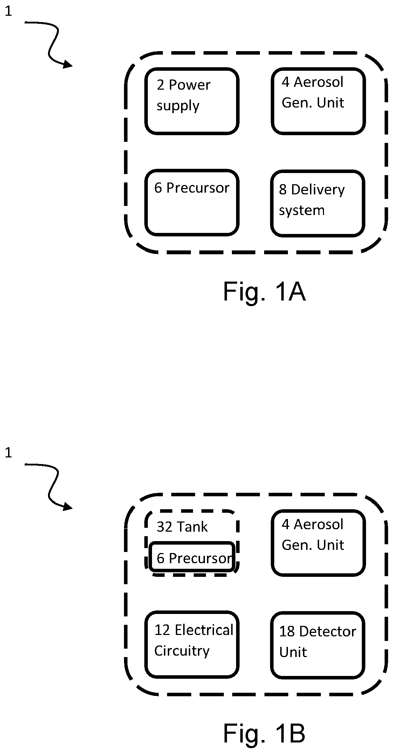

Referring to Fig. 1A, an example aerosol generating apparatus 1 includes a power supply 2, for supply of electrical energy. The aerosol generating apparatus 1 includes an aerosol generating unit 4 that is driven by the power supply 2. The power supply 2 may include an electric power supply in the form of a battery and/or an electrical connection to an external power source. The aerosol generating apparatus 1 includes a precursor 6, which in use is aerosolised by the aerosol generating unit 4 to generate an aerosol. The apparatus 2 includes a delivery system 8 for delivery of the aerosol to a user.

Electrical circuitry (not shown in figure 1) may be implemented to control the interoperability of the power supply 4 and aerosol generating unit 6.

In variant examples, which are not illustrated, the power supply 2 may be omitted since, e.g., an aerosol generating unit implemented as an atomiser with flow expansion may not require a power supply.

Referring to Fig. 1B, a further example includes an aerosol generating unit 4 that can be driven by the power supply 2 described in connection with the example of Fig. 1A. The aerosol generating apparatus 1 includes a detector unit 18 which is configured to detect a puff and to generate a puff signal as long as the puff is detected. The aerosol generating apparatus 1 includes an electrical circuitry 12 which is configured start the operation of the aerosol generating unit 4 upon receiving the puff signal and end the operation of the aerosol generating unit 4 during the reception of the puff signal. Examples of the control of the aerosol generating unit 4 are described below. The aerosol generating apparatus 1 may include storage portion implemented here as a tank 32 which is configured to store the precursor 6.

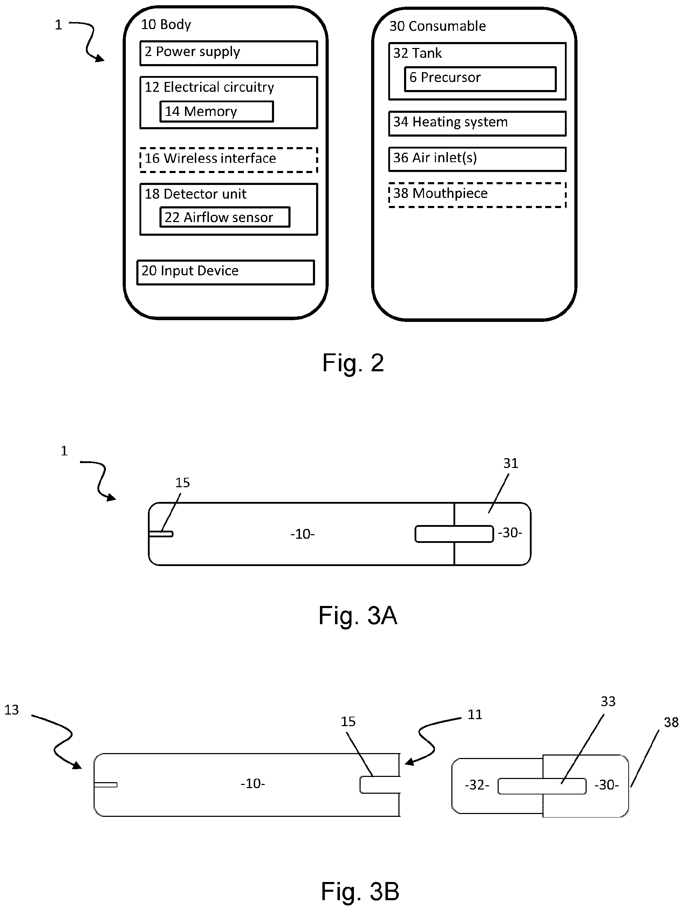

Fig. 2 shows an implementation of the aerosol generating apparatus 1 of Figs. 1A and/or 1B, where the aerosol generating apparatus 1 is configured to generate aerosol from a liquid precursor.

In this example, the apparatus 1 includes a device body 10 and a consumable 30.

In this example, the body 10 includes the power supply 4. The body may additionally include any one or more of electrical circuitry 12, a memory 14, a wireless interface 16, a detector unit 18 (e.g., including an airflow sensor 22), an input device 20, an airflow sensor 22, and one or more other components.

The electrical circuitry 12 may include a processing resource for controlling one or more operations of the body 10 and consumable 30, e.g., based on instructions stored in the memory 14. The memory 14 may be a part or portion of the electrical circuitry 12.

The wireless interface 16 may be configured to communicate wirelessly with an external (e.g., mobile) device, e.g. via Bluetooth.

The other component(s) may include one or more user interface devices configured to convey information to a user and/or a charging port, for example (see for example Fig. 3). The other component(s) may include a user interface or display for displaying various information about the aerosol generating apparatus 1.

The consumable 30 includes a storage portion implemented here as a tank 32 which stores the liquid precursor 6 (e.g., e-liquid). The consumable 30 also includes a heating system 34 (an example of the aerosol generating unit 4), one or more air inlets 36, and a mouthpiece 38. The consumable 30 may include one or more other components 40.

The body 10 and consumable 30 may each include a respective electrical interface (not shown) to provide an electrical connection between one or more components of the body 10 with one or more components of the consumable 30. In this way, electrical power can be supplied to components (e.g. the heating system 34) of the consumable 30, without the consumable 30 needing to have its own power supply.

In use, a user may activate the aerosol generating apparatus 1 when inhaling through the mouthpiece 38, i.e. when performing a puff. The puff, performed by the user, may initiate a flow through a flow path in the consumable 30 which extends from the air inlet(s) 34 to the mouthpiece 38 via a region in proximity to the heating system 34.

Operation of the aerosol generating apparatus 1 may be initiated, for example, by the airflow sensor 22 in the body 10 which detects airflow in the aerosol generating apparatus 1 (e.g. caused by a user inhaling through the mouthpiece), and/or by actuation of the input device 20 included in the body 10. During operation, the electrical circuitry 12 (e.g. under control of the processing resource) may supply electrical energy from the power supply 2 to the heating system 34 which may cause the heating system 32 to heat liquid precursor 6 drawn from the tank to produce an aerosol which is carried by the flow out of the mouthpiece 38.

In some examples, the heating system 34 may include a heating filament and a wick, wherein a first portion of the wick extends into the tank 32 in order to draw liquid precursor 6 out from the tank 32, wherein the heating filament coils around a second portion of the wick located outside the tank 32. The heating filament may be configured to heat up liquid precursor 6 drawn out of the tank 32 by the wick to produce the aerosol.

In this example, the aerosol generating unit 4 is provided by the above-described heating system 34 and the delivery system 8 is provided by the above-described flow path and mouthpiece 38.

In variant embodiments (not shown), any one or more of the precursor 6, heating system 34, air inlet(s) 36 and mouthpiece 38, may be included in the body 10. For example, the mouthpiece 36 may be included in the body 10 with the precursor 6 and heating system 32 arranged as a separable cartomizer. Figs. 3A and 3B show an example implementation of the aerosol generating device 1 of Fig. 2. In this example, the consumable 30 is implemented as a capsule/pod, which is shown in Fig. 3A as being physically coupled to the body 10, and is shown in Fig. 3B as being decoupled from the body 10.

In this example, the body 10 and the consumable 30 are configured to be physically coupled together by pushing the consumable 30 into an aperture in a top end 11 the body 10, with the consumable 30 being retained in the aperture via an interference fit.

In other examples (not shown), the body 10 and the consumable 30 could be physically coupled together in other ways, e.g. by screwing one onto the other, through a bayonet fitting, or through a snap engagement mechanism, for example.

The body 10 also includes a charging port (not shown) at a bottom end 13 of the body 10.

The body 10 also includes a user interface device configured to convey information to a user. Here, the user interface device is implemented as a light 15, which may e.g. be configured to illuminate when the apparatus 1 is activated. Other user interface devices are possible, e.g. to convey information haptically or audibly to a user.

In this example, the consumable 30 has an opaque cap 31, a translucent tank 32 and a translucent window 33. When the consumable 30 is physically coupled to the body 10 as shown in Fig. 3A, only the cap 31 and window 33 can be seen, with the tank 32 being obscured from view by the body 10. The body 10 includes a slot 15 to accommodate the window 33. The window 33 is configured to allow the amount of liquid precursor 6 in the tank 32 to be visually assessed, even when the consumable 30 is physically coupled to the body 10.

Turning back to Fig. 2, the detector unit 18 may include an airflow sensor 22 which is configured to measure a flow rate of the aerosol and/or of air flowing through aerosol generating apparatus. The airflow sensor 22 is arranged in the flow path or is in fluid communication with the flow path. The airflow sensor 22 may be downstream of the air inlet 36 and upstream of the heating system 34. In this case, the airflow sensor 22 measures an air flow that is drawn in through the air inlet 36 by the user during a puff. The airflow sensor 22 may be arranged in the body 10.

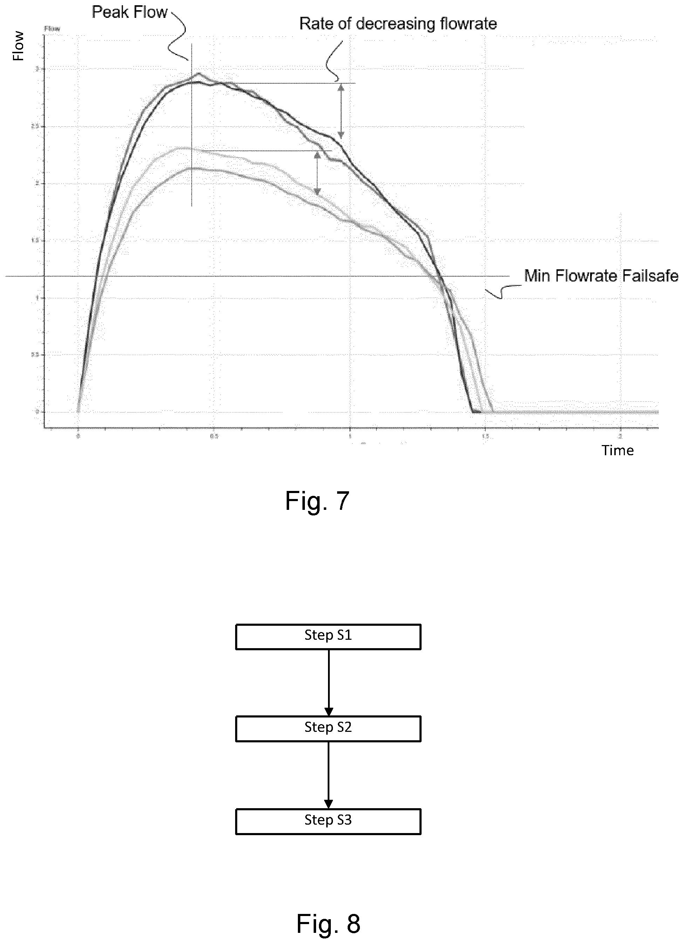

The airflow sensor 22 may be configured to measure the current airflow at each or many time points during a puff of the user (exemplary graphs of average flow rates of various users during a puff are shown in Fig. 7). For example, the airflow sensor 22 may be configured to periodically measure the airflow, e.g. every 5 ms or every 10 ms. Further, the airflow sensor 22 may be configured to convert the measured airflow into an electrical or electronic signal indicative of the strength of the airflow and send the signal to the electrical circuitry 12. The electrical circuitry 12 may be configured to calculate a peak of the flow rate or a change in flow rate.

Fig. 7 shows various flow rates, each graph representing an average flow rate (averaged over a plurality of puffs and a plurality of users) for different types of aerosol generating apparatuses 1. It is immediately apparent from Fig. 7, that the respective flow rate graphs are similar to each other in that, at the beginning of the puff, there is a steep increase in the flow rate until a peak flow, and then there is a continuous decrease in the flow rate. At first, the decrease in the flow rate is slow but, at the end of the puff, the decrease in the flow rate is steep. Thus, if the rate of the decrease in the flow rate is above a certain threshold, this can be reliably used to determine the end of the puff. Further, the time interval between the peak of the flow rate and the end of the puff is approximately equal for the various flow rate profiles. Thus, the peak of the flow rate is a good indicator for the end of the puff.

The electrical circuitry 12 may be configured to estimate the end of the puff by determining/monitoring the peak in the flow rate and the rate of decrease in the flow rate. For example, if the rate of decrease in the flow rate is above a certain threshold, this can be used as an indication for the expected end of the puff. In some examples, the end of the puff is estimated to be a predetermined time period after the peak of the flow rate and/or the point of time when the rate of the decrease in the flow rate is above the predetermined threshold. Then, the electrical circuitry 12 is configured is configured to end the operation of the heating system 34 at the expected end of the puff minus a preset timeslot. So, the electrical circuitry 12 ends the operation of the heating system 34 although the user still draws on the aerosol generating system 1. In other words, the electrical circuitry 12 terminates the operation of the heating system 34 while it still receives the puff signal. So, the electrical circuitry 12 ends the operation of the heating system 34 prior to the end of the puff.

The electrical circuitry 12 may implement the general concept of ending the operation of the heating system 34 by estimating the expected end of the puff minus the preset timeslot by ending the operation of the heating system 34 after a predetermined time period after the peak of the flow rate and/or after the rate of decrease in the flow rate is above a predetermined threshold. So, the predetermined time period after the peak of the flow rate and/or the predetermined threshold are equivalent to the estimation of the expected end of the puff minus the timeslot because all these three approaches rely on the fact that the end of the puff is estimated and the electrical circuitry 12 ends the operation of the heating system 34 prior to the end of the puff.

The input device 20 may include switches, dials, buttons, and/or a touchscreen which may be used by the user from manually setting the preset timeslot, the predetermined time period after the peak of the flow rate, and/or the predetermined threshold with regard to the rate of decrease in the flow rate. The switches, dials, buttons, and/or a touchscreen of the input device 20 may be arranged on an outer surface of the body 10 and/or the consumable 30.

Alternatively, the electrical circuitry 12 may be configured to automatically calculate or determine the predetermined time period and/or the predetermined threshold by calculating an average value over the last n puffs (n being an integer > 1, e.g. 5, 10, 15, etc) and thereby estimating the predetermined time period and/or the predetermined threshold by calculating the expected end of the puff minus the preset timeslot. For example, the electrical circuitry 12 calculates the time interval between the peak of the flow rate and the expected end of the puff and sets the predetermined time period as this time interval minus the preset timeslot. In another example, the electrical circuitry calculates the predetermined threshold as that value of the rate of the decrease in the flow rate at time point of the expected end of the puff minus the predetermined timeslot.

In a further example, the electrical circuitry 12 includes a learning mode which may be activated by using the input device 20. In the learning mode, the user draws a plurality of puffs without the heat system 34 being activated. Thus, in the learning mode, the electrical circuitry 12 solely records the flow rate of the puffs and generates an average flow rate for setting the predetermined time period and/or the predetermined threshold as outlined above.

The electrical circuitry 12 may implement a failsafe in that the electrical circuitry 12 only ends the operation of the heating system 34 when the flow rate is below a predetermined level (see horizontal line in Fig. 7).

In a further exemplary alternative, the predetermined threshold, the predetermined time period, the average puff length and/or the predetermined timeslot may be factory settings which optionally cannot be changed by the user. This reflects the fact that these values are approximately constant for all users and all types of aerosol generating apparatuses 1.

In a further example, the detector unit 18 includes a pressure sensor (not shown in the figures) which generates the puff signal (indicative of a puff of the user) if the measured pressure is below a predetermined pressure threshold. The predetermined pressure threshold may be a factory setting. The pressure sensor may be arranged at the same location as flow sensor 22. The pressure sensor may generate the puff signal if the measured pressure is below the predetermined pressure threshold. Thus, the initial receipt of the puff signal from the pressure sensor by the electrical circuitry 12 corresponds to the start of the puff. The electrical circuitry 12 may end the operation of the heating system 34 after predetermined time interval after the initial receipt of the puff signal. Here again, the predetermined time interval may be set so that it corresponds to the estimated end of the puff minus the timeslot. The predetermined time interval may be a factory setting or can be changed as described in connection with the airflow sensor 22 (e.g. by recording an average puff length by the electrical circuitry 12 or manually setting the predetermined time interval using the input device 20).

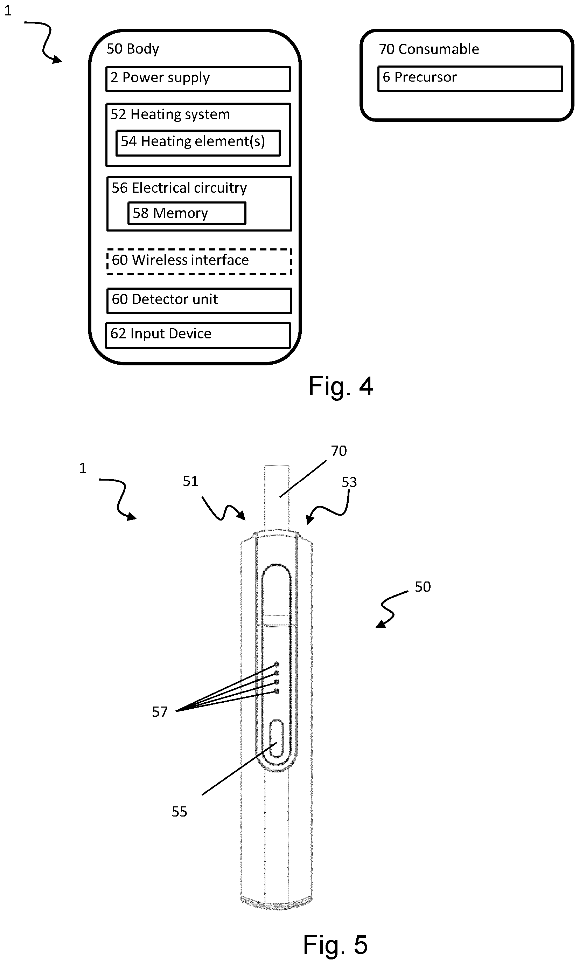

Fig. 4 shows an implementation of the apparatus 1 of Fig. 1, where the aerosol generating apparatus 1 is configured to generate aerosol by a-heat not-burn process.

In this example, the aerosol generating apparatus 1 includes a device body 50 and a consumable 70. In this example, the body 50 includes the power supply 4 and a heating system 52. The heating system 54 includes at least one heating element 54. The body may additionally include any one or more of electrical circuitry 56, a memory 58, a wireless interface 60, a detector unit 60, an input device 62, and one or more other components.

The electrical circuitry 56 may include a processing resource for controlling one or more operations of the body 50, e.g. based on instructions stored in the memory 58.

The wireless interface 60 may be configured to communicate wirelessly with an external (e.g. mobile) device, e.g. via Bluetooth.

The other component(s) 62 may include an actuator, one or more user interface devices configured to convey information to a user and/or a charging port, for example (see e.g. Fig. 5).

The body 50 is configured to engage with the consumable 70 such that the at least one heating element 54 of the heating system 52 (an example of the aerosol generating unit 4) penetrates into the solid precursor 6 of the consumable. In use, a user may activate the aerosol generating apparatus 1 to cause the heating system 52 of the body 50 to cause the at least one heating element 54 to heat the solid precursor 6 of the consumable (without combusting it) by conductive heat transfer, to generate an aerosol which is inhaled by the user.

Fig. 5 shows an example implementation of the aerosol generating device 1 of Fig. 4.

As depicted in Fig. 5, the consumable 70 is implemented as a stick, which is engaged with the body 50 by inserting the stick into an aperture at a top end 53 of the body 50, which causes the at least one heating element 54 of the heating system 52 to penetrate into the solid precursor 6.

The consumable 70 includes the solid precursor 6 proximal to the body 50, and a filter distal to the body 50. The filter serves as the mouthpiece of the consumable 70 and thus the apparatus 1 as a whole. The solid precursor 6 may be a reconstituted tobacco formulation.

In this example, the at least one heating element 54 is a rod-shaped element with a circular transverse profile. Other heating element shapes are possible, e.g. the at least one heating element may be blade-shaped (with a rectangular transverse profile) or tube-shaped (e.g. with a hollow transverse profile). In this example, the body 50 includes a cap 51. In use the cap 51 is engaged at a top end 53 of the body 50. Although not apparent from Fig. 5, the cap 51 is moveable relative to the body 50. In particular, the cap 51 is slidable and can slide along a longitudinal axis of the body 50.

The body 50 also includes an actuator 55 on an outer surface of the body 50. The actuator 55 is an example of the input device 62. In this example, the actuator 55 has the form of a button.

The body 50 also includes a user interface device configured to convey information to a user. Here, the user interface device is implemented as a plurality of lights 57, which may e.g. be configured to illuminate when the apparatus 1 is activated and/or to indicate a charging state of the power supply 4. Other user interface devices are possible, e.g. to convey information haptically or audibly to a user.

The body may also include an airflow sensor which detects airflow in the aerosol generating apparatus 1 (e.g. caused by a user inhaling through the consumable 70). This may be used to count puffs, for example.

In this example, the consumable 70 includes a flow path which transmits aerosol generated by the at least one heating element 54 to the mouthpiece of the consumable.

In this example, the aerosol generating unit 4 is provided by the above-described heating system 52 and the delivery system 8 is provided by the above-described flow path and mouthpiece of the consumable 70.

In an example, a puff may not be determined by using the airflow sensor or the air pressure sensor but the detector unt 60 is configured to measure the power that is supplied to the heating system 52. For example, the detector unit 60 may be configured to measure the current supplied to the heating system 52 from the power supply 2, the voltage of the power supply 2, and/or a resistance of the heating system 52. The detector unit 60 may be configured to calculate the power supplied to the heating system 52 based on the measured values. Parts of the detector unit 60 may be implemented by the electrical circuitry 56.

The electrical circuitry 56 may be configured to determine a puff based on the power supplied to the heating system 52. For example, the electrical circuitry 56 may control the heating system 52 such that a temperature generated by the heating system 52 is constant. In the absence of a puff, the power supply for maintaining this temperature can be approximated to be constant. In case of a puff, the airflow cools the heating system 52 so that more power for maintaining the temperature of the heating system 52 is required. So, an increase in the power supplied to the heating system 52, optionally over a predetermined period of time, indicates the presence of a puff. Using one or more of the methods as described above, the electrical circuitry 56 may be configured to end the operation of the heating system 52 before the end of the puff.

In another example, the electrical circuitry 56 is configured to control the heating system 52 by pulse width modulation (PWM). The detector unit 60 may be configured to determine the ratio of on-times to off-times of the PWM. A particular ratio of on-times to off-times may correspond to the power supply for maintaining the temperature of the heating system 52 when the user does not draw on the aerosol generating apparatus 1. An increase in the ratio on-times to off-times compared to the particular ratio of on-times to off-times may indicate the presence of a puff. A return to the particular ratio of on-times to off-times may indicate the end of the puff. In this way, the detector unit 60 may be configured to determine the start and the end of a puff. The detector unit 60 may be a part or portion of the electrical circuitry 56 in this example. The electrical circuitry 56 may end the operation of the heating system 52 before the end of the puff using the above-described exemplary methods.

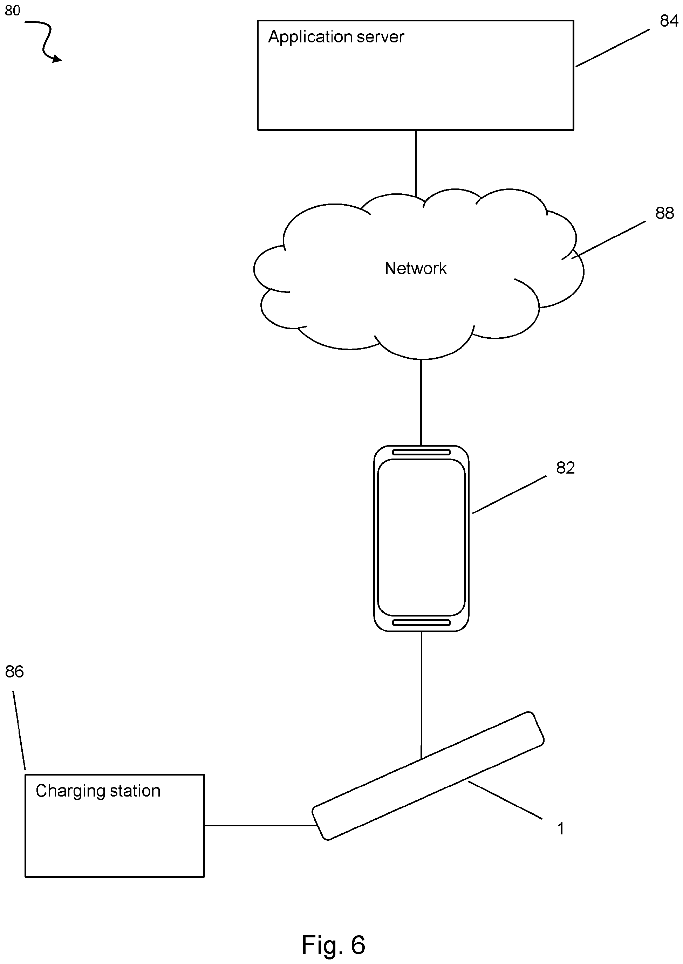

Fig. 6 shows an example system 80 for managing an aerosol generating apparatus 1, such as those described above with reference to any of Figs. 1-5.

The system 80 as shown in Fig. 1 includes a mobile device 82, an application server 84, an optional charging station 86, as well as the aerosol generating apparatus 1.

In this example, aerosol generating apparatus 1 is configured to communicate wirelessly, e.g. via Bluetooth^{™}, with an application (or "app") installed on the mobile device 2, via a wireless interface included in the aerosol generating apparatus 1 and via a wireless interface included in the mobile device 82. The mobile device 82 may be a mobile phone, for example. The application on the mobile phone is configured to communicate with the application server 84, via a network 88. The application server 84 may utilise cloud storage, for example. The network 88 may include a cellular network and/or the internet.

In other examples, the aerosol generating apparatus 1 may be configured to communicate with the application server 84 via a connection that does not involve the mobile device 82, e.g. via a narrowband internet of things ("NB-loT") or satellite connection. In some examples, the mobile device 82 may be omitted from the system 80.

A skilled person would readily appreciate that the mobile device 82 may be configured to communicate via the network 88 according to various communication channels, preferably a wireless communication channel such as via a cellular network (e.g. according to a standard protocol, such as 3G or 4G) or via a Wi-Fi network.

The app installed on the mobile device 82 and the application server 84 may be configured to assist a user with managing their aerosol generating apparatus 1, based on information communicated between the aerosol generating apparatus 1 and the app, information communicated directly between the aerosol generating apparatus 1 and the application server 84, and/or information communicated between the app and the application server 84. For example, the app installed on the mobile device 82 may be used to change or adjust the preset timeslot, the average puff length, the predetermined time period, and/or the predetermined threshold.

The charging station 86 (if present) may be configured to charge (and optionally communicate with) the aerosol generating apparatus 1, via a charging port on the aerosol generating apparatus 1. The charging port on the smoking substitute device 10 may be a USB port, for example, which may allow the aerosol generating apparatus 1 to be charged by any USB-compatible device capable of delivering power to the aerosol generating apparatus 1 via a suitable USB cable (in this case the USB-compatible device would be acting as the charging station 86). Alternatively, the charging station could be a docking station specifically configured to dock with the aerosol generating apparatus 1 and charge the aerosol generating apparatus 1via the charging port on the aerosol generating apparatus 1.

Referring to Fig. 8, a method for controlling the aerosol generating apparatus 1 for delivering an aerosol to a user for inhalation by the user is now described. The method may be executed by the various examples of the aerosol generating apparatus 1 described above.

In step S1, the puff of a user (in particular a start of the puff is detected). This may be executed by the detection unit 18, 56. In one example, as long as the puff is detected, a puff signal is generated, e.g. by the detection unit 18, 56. The puff signal may be sent to the electrical circuitry 12, 56.

In step S2, upon the start of the generation of the puff signal (e.g. when the user starts inhaling), the heating system 34, 52 is operated for generating the aerosol from the precursor 6. For example, an operation signal is generated by the electrical circuitry 12, 56 upon receiving the puff signal. The operation signal may be indicative to start the operation of the heating system 34, 52. In an example, the operation signal may be sent to a switch controlling the power supply from the power supply 2 to the heating system 34, 52.

In step S3, the operation of the heating system 34, 52 is ended before the puff ends. In one embodiment, the operation of the heating system 34, 52 is ended when the puff signal is still being generated. One or more of the exemplary methods for determining when to the operation of the heating system 34, 52 can be employed.

## Claims

1. An aerosol generating apparatus for delivering an aerosol to a user, comprising
an aerosol generating unit (4) configured to generate the aerosol from a precursor,
a detector unit (18, 60) configured to detect a start of a puff and to generate a corresponding puff signal, and
an electrical circuitry (12, 56) configured to
start the operation of the aerosol generating unit (4) upon receiving the puff signal,
end the operation of the aerosol generating unit (4) before the puff ends.

2. The aerosol generating apparatus of claim 1, wherein the detector unit (18, 60) includes an airflow sensor (22) for measuring a flow rate of the aerosol and/or of air flowing through aerosol generating apparatus (1),
wherein the electrical circuitry (12, 56) is further configured to determine an expected end of the puff based on the determined flow rate, and
wherein the electrical circuitry (12, 56) is configured to end the operation of the aerosol generating unit (4) prior to the expected end of the puff.

3. The aerosol generating apparatus of claim 2, wherein the electrical circuitry (12, 56) is further configured to determine a peak of the flow rate and end the operation of the aerosol generating unit (4) at a predetermined time period after the peak of the flow rate.

4. The aerosol generating apparatus of claims 2 or 3, wherein the electrical circuitry (12, 56) is further configured to determine a rate of decrease in the flow rate and to end the operation of the aerosol generating unit (4) after the rate of decrease in the flow rate is above a predetermined threshold.

5. The aerosol generating apparatus of claims 3 or 4, wherein the predetermined time period and/or the predetermined threshold are pre-stored values, and/or the electrical circuitry (12, 56) is further configured to calculate a rolling average of the flow rate over the last n puffs for setting the predetermined time period and/or the predetermined threshold, n being an integer > 1.

6. The aerosol generating apparatus of any one of the claims 2 to 5, wherein the electrical circuitry (12, 56) is further configured to only end the operation of the aerosol generating unit (4) if the flow rate is below a predetermined level.

7. The aerosol generating apparatus of claims 1 and 2, wherein the detector unit (18, 60) includes a pressure sensor configured to generate the puff signal when a pressure of the aerosol and/or an air flowing through aerosol generating apparatus (1) is below a predetermined pressure threshold,
wherein the electrical circuitry (12, 56) is further configured to end the operation of the aerosol generating unit (4) after a predetermined time interval after receiving the puff signal.

8. The aerosol generating apparatus of any preceding claim, wherein the detector unit (18, 60) is further configured to determine the power supplied to the aerosol generating unit (4) and generate the puff signal based on the power supplied to the aerosol generating unit (4).

9. The aerosol generating apparatus of claim 8, wherein the electrical circuitry (12, 56) is configured to control the power supplied to the aerosol generating unit (4) by using pulse width modulation (PWM), wherein the detector unit (18, 60) is configured generate the puff signal based on a ratio of on-times compared to off-times in the PWM.

10. The aerosol generating apparatus of any one of the claims 7 to 9, wherein the electrical circuitry (12, 56) is configured to set the predetermined time interval as an average puff length minus a preset timeslot.

11. The aerosol generating apparatus of claim 10, wherein the electrical circuitry (12, 56) is configured to set the average puff length by averaging a plurality of puff in a learning mode and/or by calculating a rolling average of the puff length over the last n puffs, n being an integer > 1.

12. The aerosol generating apparatus of claims 10 or 11, further comprising an input device (20, 62) for manually changing the preset time interval and/or the timeslot.

13. A method of controlling an aerosol generating apparatus (1) for delivering an aerosol to a user, comprising the steps of
detecting a start of a puff of the user and generating a corresponding puff signal,
starting the operation of an aerosol generating unit (4) for generating the aerosol from the precursor as soon as the puff signal is generated, and
ending the operation of the aerosol generating unit (4) before the puff ends.

14. The method of claim 13, wherein the step of detecting the puff includes measuring a flow rate of the aerosol and/or of air flowing through aerosol generating apparatus (1),
wherein the method further comprises determining an expected end of the puff based on the determined flow rate and ending the operation of the aerosol generating unit (4) prior to the expected end of the puff.

15. An electrical circuitry for an aerosol generating apparatus (1) for delivering an aerosol to a user, the electrical circuitry (12, 56) being configured to execute the following steps:
receiving a puff signal indicating a start of a puff,
generating an operation signal for starting the operation of an aerosol generating unit (4) for generating the aerosol from the precursor upon receiving the puff signal, and
ending the generation of the operation signal before the puff ends.
